**Europäisches Patentamt**

**European Patent Office**

**Office européen des brevets**

(19)

(11) Veröffentlichungsnummer : **0 531 863 B1**

(12)

# EUROPÄISCHE PATENTSCHRIFT

(45) Veröffentlichungstag der Patentschrift :
**27.04.94 Patentblatt 94/17**

(51) Int. Cl.$^5$ : **C07C 303/44, C07C 309/47**

(21) Anmeldenummer : **92114909.2**

(22) Anmeldetag : **01.09.92**

(54) Verfahren zur Isolierung von 2-Naphthylamin-1,5-disulfonsäure.

(30) Priorität : **12.09.91 DE 4130333**

(43) Veröffentlichungstag der Anmeldung :
**17.03.93 Patentblatt 93/11**

(45) Bekanntmachung des Hinweises auf die
Patenterteilung :
**27.04.94 Patentblatt 94/17**

(84) Benannte Vertragsstaaten :
**CH DE GB IT LI**

(56) Entgegenhaltungen :
**EP-A- 0 037 511**
**EP-A- 0 074 024**
**EP-A- 0 138 755**
**F. MUTH ' "Methoden zur Herstellung und Um-**
**wandlung aromatischer Sulfonsäuren" in ME-**
**THODEN DER ORGANISCHEN CHEMIE**
**(HOUBEN-WEYL), 4. Aufl., Band IX' 1955 ,**
**GEORG THIEME VERLAG , STUTTGART**

(73) Patentinhaber : **BAYER AG**
**D-51368 Leverkusen (DE)**

(72) Erfinder : **Behre, Horst, Dr.**
**Zur alten Linde 12**
**W-5068 Odenthal-Eikamp (DE)**
Erfinder : **Blank, Heinz-Ulrich, Dr.**
**Am Geusfelde 35**
**W-5068 Odenthal-Glöbusch (DE)**
Erfinder : **Heidenreich., Holger, Dr.**
**Blankenese 18**
**W-2224 Kuden (DE)**
Erfinder : **Pohl, Fritz, Dr.**
**Trischenring 7**
**W-2212 Brunsbüttel (DE)**
Erfinder : **Uhrhan, Paul, Dr.**
**Oskar-Schlemmer-Strasse 4**
**W-5090 Leverkusen (DE)**

**Beschreibung**

Die Erfindung betrifft die Isolierung von 2-Naphthylamin-1,5-disulfonsäure ("Sulfotobiassäure") aus diese Säure enthaltenden Sulfiergemischen in verbesserter Qualität.

2-Naphthylamin-1,5-disulfonsäure ist ein wichtiges Produkt für die Herstellung von Farbstoffen, insbesondere von Reaktivfarbstoffen. Sie kann aus 2-Naphthylamin-1-sulfonsäure ("Tobiassäure") durch Sulfierung in rauchender Schwefelsäure ("Oleum") mit einem $SO_3$-Gehalt von 15 bis 30 Gew.-%, z.B. bei Raumtemperatur, hergestellt werden.

Aus den entstehenden Reaktionsgemischen kann man die 2-Naphthylamin-1,5-disulfonsäure durch Verdünnen ausfällen. Diese Abscheidung der freien Säure ist mit dem Nachteil verbunden, daß die als freie Säure isolierte 2-Naphthylamin-1,5-disulfonsäure durch Zersetzungsprodukte wie 2-Naphthylamin und 2-Naphthylamin-5-sulfonsäure verunreinigt ist (s. Bucherer und Wahl, J. pr. Chemie 103, 151 (1921); "Methoden der organischen Chemie", (Houben-Weyl), Bd. 9, Georg Thieme Verlag, Stuttgart 1955, S. 481-484; Donaldson, The Chemistry and Technology of Naphthalene Compounds, Edward Arnold (Publishers) LTD., London 1957; Armstrong, Wynne, Chem. New 62, 163 (1890)) und in einer Kristallform ausfällt, in der sie erhebliche Mengen (15-20 Gew.-%) Schwefelsäure enthält. Dieser Schwefelsäuregehalt der abgetrennten 2-Naphthylamin-1,5-disulfonsäure läßt sich durch Waschen mit Wasser oder stark verdünnter Schwefelsäure infolge der hohen Löslichkeit der 2-Naphthylamin-1,5-disulfonsäure in Wasser und verdünnter Schwefelsäure nur unter hohen Ausbeuteverlusten vermindern.

Es wurde nun gefunden, daß man die bei der Ausfällung der 2-Naphtylamin-1,5-disulfonsäure in Form der freien Säure auftretenden Schwierigkeiten dadurch vermeiden kann, daß man die Ausfällung durch Verdünnen der Reaktionsgemische unter exakt definierten Bedingungen vornimmt.

Gegenstand der Erfindung ist ein Verfahren zur Isolierung von 2-Naphthylamin-1,5-disulfonsäure aus einem Reaktionsgemisch, das, bezogen auf das gesamte Reaktionsgemisch, 15 bis 35, vorzugsweise 20 bis 30 Gew.-% 2-Naphthylamin-1,5-disulfonsäure und 65 bis 85, vorzugsweise 70 bis 80 Gew.-% Schwefelsäure oder Oleum mit einem $SO_3$-Gehalt, bezogen auf Schwefelsäure + $SO_3$, von 1 bis 20, vorzugsweise 10 bis 17 Gew.-% enthält, durch (a) Verdünnen mit verdünnter Schwefelsäure und Wasser auf einen Schwefelsäuregehalt, bezogen auf Schwefelsäure und Wasser, von 20 bis 80, vorzugsweise 35 bis 55, besonders bevorzugt 40 bis 50 und insbesondere 45 bis 48 Gew.-% unter (b) Aufrechterhaltung einer Temperatur von 30 bis 80, vorzugsweise von 35 bis 70, besonders bevorzugt von 45 bis 65 und insbesondere von 54 bis 59°C in einer Weise, daß (i) das Verhältnis von zugesetzter zu ursprünglich vorhandener Schwefelsäure - berechnet als 100 %ige Schwefelsäure - mindestens 0,3, vorzugsweise mindestens 0,5, insbesondere mindestens 0,75 beträgt und (ii) der Schwefelsäuregehalt während des gesamten Verdünnungsvorgangs - ideale Vermischung vorausgesetzt - die Untergrenze des Bereichs (a) um nicht mehr als 10 % unterschreitet.

Wesentlich für das Erzielen des erfindungsgemäßen Effekts ist also - neben der Temperatur - nicht nur der Schwefelsäuregehalt am Ende des Verfahrens, sondern offenbar auch das Vermeiden von temporär zu niedrigen Schwefelsäurekonzentrationen, wie sie z.B. beim Einlaufenlassen des Reaktionsgemisches in Wasser durchlaufen werden. Erfindungsgemäß arbeitet man also vorzugsweise so, daß vom Schwefelsäuregehalt des Reaktionsgemischs aus betrachtet beim Verdünnen möglichst kein Minimum oder nur ein solches Minimum durchschritten wird, das dem Schwefelsäuregehalt-Endwert eng benachbart ist.

Grundsätzlich kann das erfindungsgemäße Verfahren so ausgeführt werden, daß man das zu verdünnende Reaktionsgemisch und Wasser in verdünnte Schwefelsäure einer Konzentration gibt, die wenigstens den anspruchsgemäß geforderten Schwefelsäuremindestgehalt besitzt.

Nach einer bevorzugten Ausführungsform werden Reaktionsgemisch, Wasser und verdünnte Schwefelsäure gleichzeitig so dosiert, daß sich die gewünschte Konzentration einstellt und während des gesamten Mischungsvorgangs praktisch konstant gehalten wird.

Anstelle frischer verdünnter Schwefelsäure kann zum Verdünnen auch die nach dem Ausfallen der 2-Naphthylamin-1,5-disulfonsäure verbleibende Schwefelsäure ("Muttersäure") eines vorhergegangenen Ansatzes verwendet werden. Solche "Muttersäure" enthält, bezogen auf die Summe von Wasser und Schwefelsäure, im allgemeinen 20 bis 80, vorzugsweise 35 bis 55, besonders bevorzugt 40 bis 50 und insbesondere 45 bis 48 Gew.-% Schwefelsäure und, bezogen auf Schwefelsäure, 0,1 bis 3,0, vorzugsweise 0,5 bis 1,5 Gew.-% 2-Naphthylamin-1,5-disulfonsäure, 0,01 bis 1,0, vorzugsweise 0,05 bis 0,2 Gew.-% 2-Naphthylamin-1,6-disulfonsäure und 0,01 bis 1,0, vorzugsweise 0,05 bis 0,2 Gew.-% 2-Naphthylamin-1,7-disulfonsäure. Es hat sich herausgestellt, daß die Verwendung von Muttersäure anstelle reiner verdünnter Schwefelsäure zu besserer Filtrierbarkeit und zu einem geringeren Schwefelsäuregehalt des isolierten Produkts führt.

Die beim Verdünnungsvorgang anfallende Wärme wird durch entsprechende Kühlung abgeführt.

Bei dem erfindungsgemäßen Verfahren fällt die 2-Naphthylamin-1,5-disulfonsäure in einer gut filtrierbaren Form an; der Gehalt an 2-Naphthylamin-1,5-disulfonsäure liegt im Bereich oberhalb von 65 Gew.-%, bezogen auf feuchtes Produkt (oberhalb von 80 Gew.-%, umgerechnet auf trockenes Produkt), und der Gehalt an Schwefelsäure, bezogen auf feuchtes Produkt, liegt zwischen 8 und 14 Gew.-% (berechnet als 100 %ige $H_2SO_4$) (zwischen 9 und 17 Gew.-%, umgerechnet auf trockenes Produkt).

Es hat sich als vorteilhaft erwiesen, die ausgefällte 2-Naphthylamin-1,5-disulfonsäure noch 0,1 bis 10, vorzugsweise 0,2 bis 5, insbesondere 0,5 bis 2 Stunden bei Temperaturen von 20 bis 55°C, vorzugsweise bei 30 bis 45°C in der verdünnten Lösung zu belassen, bevor man das Produkt abtrennt.

Das erfindungsgemäße Verfahren kann diskontinuierlich und kontinuierlich ausgeführt werden.

Die Prozentangaben in den nachfolgenden Beispielen beziehen sich auf das Gewicht.

Beispiele

Beispiel 1

|  | | |
|---|---|---|
|  | | In einem 2 Ltr.-Fünfhalskolben mit Säbel-rüher, 2 Dosiertropftrichtern, Kühler und Innenthermometer werden |
| 600 | g | 50 %ige wäßrige Schwefelsäure vorgelegt und auf 40°C erwärmt. Im Verlauf von ca. 4 Stun-den läßt man bei 40°C simultan unter Kühlung |
| 500 | g | Sulfiergemisch, das durch Umsetzung von 2-Naphthylamin-1-sulfonsäure (Tobias-Säure) mit Oleum (25 % $SO_3$-Gehalt) bei 20°C erhalten worden ist (Gehalt 23,4 %, 2-Naphthylamin-1,5-disulfonsäure; 1,5 % 2-Naphthylamin-1,6-disulfonsäure, 0,3 % 2-Naphthylamin-1,7-disulfonsäure; Rest zu 100 % Schwefelsäure, Schwefeltri-oxid, organische Nebenprodukte unbekannter Struktur) und |
| 500 | g | Wasser einlaufen. Die resultierende sehr gute sedimentierende Kristall-Suspension wird ca. 1 Stunde bei 40°C nachgerührt, unter weiterem Rühren im Verlaufe mehrerer Stunden auf ca. 20°C abgekühlt und mehrere Stunden bei 20°C nachgerührt. Das sehr gut filtrierbare Produkt wird auf einer Glas-sinternutsche abgesaugt und zur Verdrän-gung der anhaftenden Muttersäure mit |
| 125 | g | 50 %iger wäßriger Schwefelsäure gewaschen. Es werden erhalten (Mittelwert aus 6 Versuchen mit 5 Ablauf-Rückführungen) |
| 160 | g | 2-Naphthylamin-1,5-disulfonsäure feucht und |

| 1535 | g | Ablauf + Waschsäure, wovon |
| 600 | g | zurückgeführt werden. |
| | | Der durch HPLC bestimmte Gehalt der iso- |
| | | lierten Produkte beträgt durchschnittlich: |
| 69,9 | % | 2-Naphthylamin-1,5-disulfonsäure, |
| 3,2 | % | 2-Naphthylamin-1,6-disulfonsäure, |
| < 0,05 | % | 2-Naphthylamin-1-sulfonsäure, |
| < 0,05 | % | 2-Naphthylamin-5-sulfonsäure, |
| < 0,05 | % | 2-Naphthylamin-8-sulfonsäure, |
| 0,05 | % | 2-Naphthylamin-1,7-disulfonsäure, |
| 11,1 | % | Schwefelsäure und |
| 15,4 | % | Wasser |
| Σ 99,6 | % | Die Ausbeute beträgt 95 % d.Th., bezogen auf eingesetzte 2-Naphthylamin-1,5-disulfonsäure (in Form des Sulfiergemisches). |

## Beispiel 2

293 g Tobiassäure (99,5 % Gehalt) werden mit 1 240 g Oleum (25 % $SO_3$-Gehalt) unter sofortiger Abführung der Reaktionswärme bei einer Temperatur von 18-20°C sulfiert. Nach einer Verweilzeit von 1 Stunde bei 20°C hat das Reaktionsgemisch folgende Zusammensetzung:

| | | |
|---|---|---|
| 2-Naphthylamin-1,5-disulfonsäure | 24,7 | % |
| 2-Naphthylamin-1,6-disulfonsäure | 0,8 | % |
| 2-Naphthylamin-1,7-disulfonsäure | 0,3 | % |
| 2-Naphthylamin-1-sulfonsäure | 0,1 | % |
| Schwefelsäure | 61,0 | % |
| $SO_3$ | 12,2 | % |
| Σ | 100,1 | % |

1533 g des Reaktionsgemisches werden simultan und kontinuierlich mit 1580 g Wasser und mit 1840 g 50 %iger wäßriger Schwefelsäure bei 40°C versetzt. Nach ca. 24 Stunden bei 40°C wird filtriert. Man erhält 562 g eines farblosen Filterkuchens der folgenden Zusammensetzung:

| 2-Naphthylamin-1,5-disulfonsäure | 66,0 | % |
|---|---|---|
| 2-Naphthylamin-1,6-disulfonsäure | 1,2 | % |
| 2-Naphthylamin-1,7-disulfonsäure | 0,4 | % |
| 2-Naphthylamin-1-sulfonsäure | 0,1 | % |
| Wasser | 19,5 | % |
| Schwefelsäure | 13,5 | % |
| Σ | 100,2 | % |

Beispiel 3

1533 g des in Beispiel 2 bereiteten Reaktionsgemisches werden kontinuierlich und simultan mit 1580 g Wasser und 1840 g rückgeführter Muttersäure aus Beispiel 2 bei 58°C verdünnt. Nach einer Verweilzeit von 1 Stunde bei 58°C wird die partiell ausgefällte Suspension in einen weiteren Rührbehälter abgelassen und innerhalb einer Stunde auf ca. 40°C abgekühlt. Nach einer Rührzeit von weiteren 23 Stunden wird die Suspension filtriert. Man erhält 488 g eines farblosen, körnigen Filterkuchens folgender Zusammensetzung:

| 2-Naphthylamin-1,5-disulfonsäure | 75,9 | % |
|---|---|---|
| 2-Naphthylamin-1,6-disulfonsäure | 1,8 | % |
| 2-Naphthylamin-1,7-disulfonsäure | 0,4 | % |
| 2-Naphthylamin-1-sulfonsäure | 0,05 | % |
| Wasser | 13,6 | % |
| Schwefelsäure | 8,1 | % |
| Σ | 99,85 | % |

Vergleich

1533 g des in Beispiel 2 bereiteten Reaktionsgemisches werden kontinuierlich mit 1580 g Wasser bei 40°C ohne Zusatz von verdünnter Schwefelsäure verdünnt. Nach einer Verweilzeit von 24 Stunden wird die Suspension filtriert Man erhält 638 g eines farblosen filzigen Filterkuchens der folgenden Zusammensetzung:

| 2-Naphthylamin-1,5-disulfonsäure | 58,1 | % |
|---|---|---|
| 2-Naphthylamin-1,6-disulfonsäure | 1,5 | % |
| 2-Naphthylamin-1,7-disulfonsäure | 0,6 | % |
| 2-Naphthylamin-5-sulfonsäure | 0,1 | % |
| 2-Naphthylamin-1-sulfonsäure | 0,1 | % |
| Wasser | 19,5 | % |
| Schwefelsäure | 19,6 | % |
| Σ | 100,0 | % |

**Patentansprüche**

1. Verfahren zur Isolierung von 2-Naphthylamin-1,5-disulfonsäure aus einem Reaktionsgemisch, das, bezogen auf das gesamte Reaktionsgemisch, 15 bis 35 Gew.-% 2-Naphthylamin-1,5-disulfonsäure und 65 bis 85 Gew.-% Schwefelsäure oder Oleum mit einem $SO_3$-Gehalt, bezogen auf Schwefelsäure + $SO_3$, von 1 bis 20 Gew.-% enthält, durch (a) Verdünnen mit verdünnter Schwefelsäure und Wasser auf einen Schwefelsäuregehalt, bezogen auf Schwefelsäure und Wasser, von 20 bis 80 Gew.-% unter (b) Aufrechterhaltung einer Temperatur von 30 bis 80°C in einer Weise, daß (i) das Verhältnis von zugesetzter zu ursprünglich vorhandener Schwefelsäure - berechnet als 100 %ige Schwefelsäure - mindestens 0,3 beträgt und (ii) der Schwefelsäuregehalt während des gesamten Verdünnungsvorgangs - ideale Vermischung vorausgesetzt - die Untergrenze des Bereichs (a) um nicht mehr als 10 % unterschreitet.

2. Verfahren nach Anspruch 1, wonach ein Reaktionsgemisch mit einem Gehalt von 20 bis 30 Gew.-% 2-Naphthylamin-1,5-disulfonsäure eingesetzt wird.

3. Verfahren nach Anspruch 1, wonach ein Reaktionsgemisch mit einem Gehalt von 70 bis 80 Gew.-% Schwefelsäure oder Oleum eingesetzt wird.

4. Verfahren nach Anspruch 1, wonach das Oleum einen $SO_3$-Gehalt von 10 bis 17 Gew.-% besitzt.

5. Verfahren nach Anspruch 1, wonach man auf einen Schwefelsäuregehalt von 35 bis 55 Gew.-% verdünnt.

6. Verfahren nach Anspruch 1, wonach man auf einen Schwefelsäuregehalt von 40 bis 50 Gew.-% verdünnt.

7. Verfahren nach Anspruch 1, wonach man auf einen Schwefelsäuregehalt von 45 bis 48 Gew.-% verdünnt.

8. Verfahren nach Anspruch 1, wonach man in einem Temperaturbereich von 35 bis 70°C arbeitet.

9. Verfahren nach Anspruch 1, wonach das Verhältnis (i) mindestens 0,5 beträgt.

10. Verfahren nach Anspruch 1, wonach man gleichzeitig mit Wasser und verdünnter Schwefelsäure verdünnt.

11. Verfahren nach Anspruch 1, wonach man anstelle von verdünnter Schwefelsäure Muttersäure eines vorangegangenen Ansatzes verwendet.

**Claims**

1. Process for the isolation of 2-naphthylamine-1,5-disulphonic acid from a reaction mixture containing, relative to the entire reaction mixture, 15 to 35 % by weight of 2-naphthylamine-1,5-disulphonic acid and 65 to 85 % by weight of sulphuric acid or oleum having an $SO_3$ content, relative to sulphuric acid + $SO_3$, of 1 to 20 % by weight, by (a) diluting the reaction mixture with dilute sulphuric acid and water to a sulphuric acid content, relative to sulphuric acid and water, of 20 to 80 % by weight, while (b) maintaining a temperature of 30 to 80 °C in such a way that (i) the ratio of added sulphuric acid to sulphuric acid originally present - calculated as 100% pure sulphuric acid - is at least 0.3, and (ii) the sulphuric acid content during the entire dilution process - assuming ideal mixing - does not fall below the lower limit of range (a) by more than 10%.

2. Process according to Claim 1, wherein the reaction mixture used has a 2-naphthylamine-1,5-disulphonic acid content of 20 to 30% by weight.

3. Process according to Claim 1, wherein the reaction mixture used has a sulphuric acid or oleum content of 70 to 80% by weight.

4. Process according to Claim 1, wherein the oleum has an $SO_3$ content of 10 to 17% by weight.

5. Process according to Claim 1, wherein the reaction mixture is diluted to a sulphuric acid content of 35 to 55% by weight.

6. Process according to Claim 1, wherein the reaction mixture is diluted to a sulphuric acid content of 40 to 50% by weight.

7. Process according to Claim 1, wherein the reaction mixture is diluted to a sulphuric acid content of 45 to 48% by weight.

8. Process according to Claim 1, wherein the reaction is carried out in a temperature range from 35 to 70°C.

9. Process according to Claim 1, wherein the ratio (i) is at least 0.5.

10. Process according to Claim 1, wherein dilution is carried out simultaneously with water and dilute sulphuric acid.

11. Process according to Claim 1, wherein the mother acid of a previous batch is used instead of dilute sulphuric acid.


**Revendications**

1. Procédé pour isoler l'acide 2-naphtylamine-1,5-disulfonique d'un mélange de réaction qui contient, par rapport au mélange de réaction total, 15 à 35 % en poids d'acide 2-naphtylamine-1,5-disulfonique et 65 à 85 % en poids d'acide sulfurique ou d'oléum d'une teneur en $SO_3$, par rapport à l'acide sulfurique + $SO_3$, de 1 à 20 % en poids, par (a) dilution par l'acide sulfurique dilué et l'eau à une teneur en acide sulfurique, par rapport à l'acide sulfurique et l'eau, de 20 à 80 % en poids avec (b) maintien d'une température de 30 à 80°C, caractérisé en ce que (i) le rapport de l'acide sulfurique ajouté à l'acide sulfurique initialement présent - calculé en acide sulfurique à 100 % - est d'au moins 0,3 et (ii) la teneur en acide sulfurique pendant tout le processus de dilution - dans l'hypothèse d'un mélange idéal - ne diminue pas au-delà de plus de 10 % au-dessous de la limite inférieure de l'intervalle (a).

2. Procédé selon la revendication 1, dans lequel on utilise un mélange de réaction ayant une teneur en acide 3-naphtylamine-1,5-disulfonique de 20 à 30 % en poids.

3. Procédé selon la revendication 1, dans lequel on utilise un mélange de réaction ayant une teneur en acide sulfurique ou en oléum de 70 à 80 % en poids.

4. Procédé selon la revendication 1, dans lequel l'oléum a une teneur en $SO_3$ de 10 à 17 % en poids.

5. Procédé selon la revendication 1, dans lequel on dilue à une teneur en acide sulfurique de 35 à 55 % en poids.

6. Procédé selon la revendication 1, dans lequel on dilue à une teneur en acide sulfurique de 40 à 50 % en poids.

7. Procédé selon la revendication 1, dans lequel on dilue à une teneur en acide sulfurique de 45 à 48 % en poids.

8. Procédé selon la revendication 1, dans lequel on opère dans un intervalle de températures de 35 à 70°C.

9. Procédé selon la revendication 1, dans lequel le rapport (i) est d'au moins 0,5.

10. Procédé selon la revendication 1, dans lequel on dilue simultanément par l'eau et l'acide sulfurique dilué.

11. Procédé selon la revendication 1, dans lequel on utilise au lieu d'acide sulfurique dilué l'acide mère d'une charge précédente.